# EUROPEAN PATENT APPLICATION

(11) **EP 4 187 548 A1**
(43) Date of publication of application: **31.05.2023**
(21) Application number: 21211523.2
(22) Date of filing: 30.11.2021
(51) Int. Cl.: G16H 40/20

(54) **METHOD FOR ARCHIVING A SAMPLE TUBE, METHOD FOR RETRIEVING A SAMPLE TUBE AND TUBE RACK**

(71) Applicant: Beckman Coulter Inc., Brea, California 92821 (US)
(72) Inventor: PYRSAK, Sebastian, 38-300 Gorlice (PL)
(74) Representative: Müller-Boré & Partner Patentanwälte PartG mbB

(57) **Abstract**

An aspect of the present invention relates to an archiving method for archiving in a storage, in particular in a cold storage, at least one sample tube containing a biological sample using at least one tube rack, the method comprising the following steps: receiving, by a computing system, a request for archiving the at least one sample tube, placing the at least one sample tube in at least one receptacle of the at least one tube rack to receive the at least one sample tube, recording and/or updating, in a memory unit of the computing system, sample-tube-storage-data including sample tube data and/or tube rack data and/or storage data, storing the at least one tube rack in the storage. Further aspects relate to a retrieving method and a tube rack.

## Description

The present invention relates to an archiving method for archiving in a storage, in particular in a cold storage, at least one sample tube containing a biological sample using at least one tube rack, a retrieving method for retrieving at least one sample tube containing a biological sample from at least one tube rack stored in a storage, in particular a cold storage, and a tube rack for receiving at least one sample tube containing a biological sample.

Conventionally, biological sample (hereinafter referred to as "sample") tracking and positive sample identification in post-analytical manual workflows is always time-consuming and has a high risk of patient misidentification. Beside the total lab automation (TLA) processes where the workflow is always fully automated and enables users to perform automatic re-runs and/or add-ons, repeating a manual process is fully human-dependent. Whenever manual archiving is done, there is a certain involvement of staff required for the process. Similarly, this is also observed with TLA systems without automatic storages as well as with standalone sorters. To search for samples, users need to search for a particular sample in the tube rack location, but, afterwards, do not have any confirmation, besides visual, that the right sample was picked.

Thus, a problem to be solved by the present invention is to provide an improved archiving and retrieving method for one or more sample tubes.

This problem is solved by the independent claims. Specific embodiments result from the respective dependent claims.

An aspect of the present invention relates to an archiving method for archiving in a storage, in particular in a cold storage, at least one sample tube containing a biological sample using at least one tube rack, the method comprising the following steps:
receiving, by a computing system, a request for archiving the at least one sample tube,
placing the at least one sample tube in at least one receptacle of the at least one tube rack to receive the at least one sample tube,
recording and/or updating, in a memory unit of the computing system, sample-tube-storage-data including sample tube data and/or tube rack data and/or storage data, and
storing the at least one tube rack in the storage.

Advantageously, the aforementioned aspect facilitates sample tube tracking. It renders sample tube tracking less time-consuming and more reliable. Furthermore, it lowers the risk of sample and thus patient misidentification. Therefore, errors in associating a sample with a particular patient may be reduced.

In this description, the term "at least one" refers to one or two, three, four, five, six, seven, eight or even more. The term "at least one" can refer to exactly one or multiple. It can refer to a plurality. The term "at least one" refers to one or more. It can be two or more.

In this description, the term "sample-tube-storage-data" may also be referred to as "sample tube storing identification data".

The sample tube may be a container for a biological sample or a container containing a biological sample. The biological sample may be any biological substance, such as a body fluid like blood, which is to be examined in a laboratory. The storage may be a cold or cooled storage. The storage may be a closable rack comprising one or more shelves to hold and/or store the at least one tube rack. The storage may be a fridge. The storage may comprise or be one or more closable rooms having multiple racks. The at least one tube rack may be a holder or support for the at least one sample tube. The at least one tube rack may be a tube rack according to a further aspect of the present invention as described below. According to the present invention, the at least one tube rack is a tube rack comprising at least one receptacle to receive the at least one tube rack.

In one embodiment, the at least one tube rack may include two or more tube racks. Each tube rack may comprise two or more receptacles.

In a specific embodiment, the method steps may be ordered as follows:
In a first step, the request for archiving the at least one sample tube may be received by the computing system. In other words, the request may be received at, on or via the computing system. The request may be associated with a user input. The request may comprise instructions causing a processor of the computing system to perform one or more actions such as retrieving and/or recording data. The computing system may be a cloud computing platform and/or a distributed computer system. The computing system may be a networked computing system comprising a server and one or more client computers or workstations.

In a next step, the at least one sample tube to be archived may be placed in the at least one receptacle of the at least one tube rack. The at least one receptacle may for example be an opening or a support clip of the at least one tube rack. The at least one sample tube may be placed in the at least one receptacle based on the sample-tube-storage-data, in particular the sample tube data and/or the tube rack data. The placing may be based on the display of the sample-tube-storage-data, in particular the sample tube data and/or the tube rack data, by the computing system. The placing may be done manually or by a lab automation system, in particular a robotic arm.

The at least one tube rack may have one or multiple receptacles.

In a further next step, the sample-tube-storage-data including the sample tube data and/or the tube rack data and/or the storage data may be recorded or updated in the memory unit of the computing system for later use.

In a next step, in particular a final step, the at least one tube rack in which the at least one sample tube to be archived has been placed may be stored in the storage. The at least one tube rack to be stored may be transferred, manually or by a lab automation system, in particular a robotic arm, to the storage. The computing system may assign to the at least one tube rack to be stored a specific location or position of the at least one tube rack in the storage. This location or position may for example correspond to a shelf number of the storage and a tube rack number on the shelf.

The storage may store one or multiple tube racks.

Storing the at least one tube rack in the storage may include placing the at least one tube rack in the storage.

Deviating from the above, the order of the steps may be altered such that first the sample-tube-storage-data is recorded or stored or updated and then the tube may be placed in the at least one receptacle of the rack and then the rack may be placed in the storage.

Deviating from the above, the order of the steps may be altered such that first the tube may be placed in the at least one receptacle of the rack and then the rack may be placed in the storage and then the sample-tube-storage-data is recorded or stored or updated.

Deviating from the above, the order of the steps may be altered such that storing or updating the sample-tube-storage-data may be carried out simultaneously with the step of placing the sample tube in the at least one receptacle of the rack and/or storing or updating the sample-tube-storage-data may be carried out simultaneously with the step of placing the rack in the storage. In such an embodiment, storing or updating may be repeated once or twice or more often during the steps of placing the sample tube in the at least one receptacle of the rack and/or the step of placing the rack in the storage.

Advantageously, the recording and/or updating of the sample-tube-storage-data in the memory unit of the computing system allows for tracking and retrieving an archived sample tube easily, quickly and more reliable.

The recording and/or updating may be done manually by user input or automatically by the computing system after the at least one tube rack was stored in the storage. The computing system and the storage may be communicatively coupled to each other so that storing the at least one tube rack in the storage may cause the computing system to record the sample-tube-storage-data in the memory unit of the computing system. The memory unit may for example comprise or be a hard drive in a server of the computing system or the memory unit may comprise or be a cloud storage of the computing system.

In a specific embodiment, the sample tube data may include at least one of an identifier of the at least one archived sample tube, sample tube expiration data and patient-related data. The identifier may include or be an identification number or identification code of the at least one sample tube. The sample tube expiration data may include the expiration date of the biological sample contained in the at least one sample tube and/or the number of tests or processes performed on the biological sample and/or the number of times the biological sample is to be used in tests or processes. The sample tube expiration data may also include information as to how long the at least one sample tube is utmost archived. The patient-related data may include an identification number or identification code associated with a specific patient. The patient-related data may also include biological identifiers such as blood type. The patient-related data may however not contain any information about the person of the patient.

Additionally or alternatively, the tube rack data may include at least one of an identifier of the at least one stored tube rack, tube rack expiration data, patient-related data and occupation data of the at least one tube rack. The identifier may include or be an identification number or identification code of the at least one tube rack. The tube rack expiration data may include the expiration date of the biological sample contained in the at least one sample tube placed in the at least one tube rack and/or the number of tests or processes performed on said biological sample and/or the number of times the biological sample is to be used in tests or processes and/or the number of times the at least one tube rack is to be used. The tube rack expiration data may also include information as to how long the at least one tube rack is utmost stored in the storage. The patient-related data may include an identification number or identification code associated with a specific patient. The patient-related data may also include biological identifiers such as blood type. The patient-related data may however not contain any information about the person of the patient. The occupation data of the at least one tube rack may include information about which of the at least one receptacle is occupied with a sample tube or has received a sample tube. The information may include the number and/or location of the at least one occupied receptacle or of the at least one receptacle which has received a sample tube. The information may also include the coordinates with respect to the at least one tube rack of the at least one receptacle that has received a sample tube or is occupied with a sample tube. The information may also include the coordinates of the unoccupied receptacles, i.e. the receptacles which have not received a sample tube. The information may also include which of the receptacles of the at least one tube rack is occupied and which are unoccupied.

Additionally or alternatively, the storage data may include at least one of an identifier of the storage and a location of the at least one tube rack stored in the storage. The identifier may include or be an identification number or identification code of the storage. The location of the at least one tube rack stored in the storage may include the coordinates of the at least one tube rack stored in the storage. The coordinates may for example include the shelf number of the storage and the number or position of the at least one tube rack on said shelf.

Advantageously, according to this embodiment, sample tube tracking can be made more efficient, in particular the archiving method can be carried out more efficiently.

In a specific embodiment, the method may further comprise: arranging the at least one sample tube to be archived at a scanning position of an apparatus being communicatively coupled with the computing system and configured to interact with the at least one tube rack before the at least one tube rack is stored in the storage, scanning, by the apparatus, the at least one sample tube when arranged at the scanning position with respect to the apparatus, and visually indicating, based on the scan and the request, the at least one tube rack to be stored and/or the at least one receptacle of the at least one tube rack in which the at least one sample tube is to be placed and/or the storage where the at least one tube rack is to be stored.

Advantageously, according to this embodiment, the archiving method is rendered even more reliable. It can be assured that the requested sample tube is archived. Thus, errors in sample archiving can further be reduced.

According to a specific embodiment the method further comprises positioning the at least one tube rack on a holding unit of the apparatus, wherein the visually indicating includes illuminating at least one of the receptacles of the tube rack positioned on the apparatus by at least one light emitting diode.

Advantageously, according to this embodiment, the method is even more reliable since the receptacle intended to receive a tube is highlighted by illumination of a corresponding LED. Thus, a user or a robot placing the tube in the receptacle has clear guidance and may reliably recognize and determine the receptacle among all the receptacles of the tube rack.

The apparatus may be coupled by means of a network, wired or wireless, to the computing system. The apparatus may be configured as the apparatus described by the Applicant in European patent application No. 21 190 791.0, which is hereby incorporated by reference. The apparatus may comprise or be a computing device.

The apparatus may be configured to interact with a tube rack, the tube rack comprising a data storage element, and the apparatus may comprise: a scanning unit configured to scan the at least one sample tube when the at least one sample tube is at the scanning position with respect to the scanning unit; a detection unit comprising at least one sensor configured to detect the presence of the at least one sample tube in the at least one tube rack if the tube rack is at a detection position with respect to the at least one sensor; a computing unit configured to write data to the data storage element when the at least one sensor detects the presence of the sample tube in the tube rack.

The at least one tube rack may comprise a data storage element, i.e. an element configured to store data, in particular digital data. The data storage element may be built-in, i.e. it may be permanently and securely integrated in the at least one tube rack. The data storage element may be a machine-readable and -writable memory, e.g. a memory chip. Exemplarily, the memory chip may be part of a radio-frequency identification (RFID) tag. In this case, the whole RFID tag, i.e. including the antenna and the substrate, may be comprised in the at least one tube rack. The data storage element may have an identifier for the tube rack stored thereon, e.g. a globally unique identifier (GUID).

The apparatus may be configured to interact with the at least one tube rack. The interaction may occur through contact between (a part of) the apparatus and (a part of) the at least one tube rack, e.g. the apparatus may fittingly accommodate the at least one tube rack, and/or may be contactless, e.g. a short-range wireless communication such as Bluetooth or near-field communication (NFC).

The apparatus may comprise a scanning unit for scanning the at least one sample tube. The scanning unit may be a data reading unit configured to read data from the at least one sample tube when the sample tube is arranged at the scanning position with respect to the scanning unit. The scanning position may be a physical location defined in relation to the scanning unit and it may be defined such that it enables the scanning unit to access data from the at least one sample tube. The scanning position may be at a given distance from the scanning unit. The at least one sample tube may comprise an accessible and rewritable data storage element.

The scanning unit may be a camera. The camera may for example be configured to recognize the at least one sample tube at the scanning position by means of a label having a bar code, a QR code or an identification number. Scanning, by the apparatus, may include recognizing by the camera the at least one sample tube when arranged in the scanning position. The scanning unit may also be a RFID-scanner or a NFC-enabled device. The label may be a RFID tag.

The data scanned or read from the at least one sample tube or wrote to the at least one sample tube may correspond to the sample tube data.

The step of visually indicating may include illuminating, by at least one light emitting diode integrated in the apparatus and/or the at least one tube rack and/or the storage, the at least one receptacle and/or the at least one tube rack and/or the storage. Visually indicating the storage may include visually indicating the location, i.e. for example the shelf and position of the at least one tube rack to be stored on the shelf, of the at least one tube rack to be stored in the storage. Visually indicating may also include displaying, via a display screen of the apparatus and/or of the computing system, the at least one receptacle and/or the at least one tube rack and/or the storage, in particular the location in the storage. The visual indication may correspond to the request, i.e. which sample tube is to be archived, and the scan by the scanning unit, i.e. which sample tube has been scanned or recognized by the scanning unit of the apparatus. The visual indication may only occur if the request and the scan correspond to each other. In other words, by scanning, the apparatus may confirm whether the at least one sample tube to be archived according to the request was picked and is going to be archived.

The additional method steps of this embodiment may be performed in the following order: arranging, scanning and visually indicating. These method steps may be performed before the at least one sample tube is placed in the at least one receptacle of the at least one tube rack.

Furthermore, the computing system may receive user input as to the location in the storage where the at least one tube rack is stored. Additionally or alternatively, the computing system may receive user input to record the sample-tube-storage-data. The user input may include touch input and/or voice input and/or keyboard input. After placing the at least one sample tube to be archived according to the request in the at least one tube rack and storing the at least one tube rack in the storage, the sample-tube-storage-data already existing in the memory unit may be updated manually or by the computing system.

The method may further include the step of placing the at least one tube rack in the apparatus before storing the at least one tube rack in the storage.

Advantageously, thereby, the archiving method is rendered even more reliable. It can be assured that the requested sample tube is archived. Thus, errors in sample archiving can further be reduced.

In a specific embodiment, the computing system and/or the apparatus may comprise a display screen. The request for archiving may be received via the computing system and/or the apparatus and displayed on the display screen. Further, the computing system and/or the apparatus may comprise or be a mobile device, in particular a tablet PC or a smartphone or the like. In other words, a user of the computing system and/or the apparatus may manually provide a request for archiving of a sample. In one example, the request may be provided by means of a display screen, e.g. wherein the display screen is a touch sensitive display, that is a touch screen. In another example, the request may be provided using a physical keyboard and/or a mouse and/or voice commands. The above also applies for a request for retrieving a sample.

Advantageously, according to this embodiment, the archiving method and thus sample tracking may be facilitated and/or made more user-friendly. Moreover, the archiving method may be made even more reliable.

The mobile device may be coupled to the computing system by means of a network communication. The computing system and the mobile device may be coupled by a Wi-Fi or Bluetooth connection. The mobile device may be mounted to the computing system. The mobile device may be coupled to the apparatus by means of a network communication. The apparatus and the mobile device may be coupled by a WLAN or Bluetooth connection or by a wired connection. The mobile device may be mounted to the apparatus. The display screen may be integrated in the computing system and/or the apparatus. The display screen may be mounted to the computing system and/or the apparatus. The mobile device may be attached to a device holder.

In a specific embodiment, the visually indicating may include: illuminating the at least one tube rack to be stored and/or the at least one receptacle of the at least one tube rack in which the at least one sample tube is to be placed and/or the storage where the at least one tube rack is to be stored, and/or displaying the sample tube data and/or the tube rack data and/or the storage data.

Advantageously, according to this embodiment, for instance, the storage may be illuminated by a light emitting diode so that it is clearly visible for a user where to store the at least one tube rack. Furthermore, the storage data, i.e. the location of the at least one tube rack in the storage, may for example be displayed to the user. Thereby, a sample tube may be archived and tracked more reliable.

The step of illuminating may be performed by at least one light emitting diode integrated in or mounted to the apparatus and/or the at least one tube rack and/or the storage. The sample tube data and/or the tube rack data and/or the storage data may be displayed on a display screen of the computing system and/or the apparatus. The illuminating the storage may include illuminating, e.g. by a light emitting diode, the location or position where the at least one tube rack is to be stored in the storage.

In a specific embodiment, the method may further comprise: indicating, by the computing system, based on the sample-tube-storage-data, that the at least one archived sample tube and/or the at least one stored tube rack and/or the biological sample contained in the at least one archived sample tube is expired, and/or the at least one archived sample tube and/or the at least one stored tube rack is to be retrieved from the storage.

Advantageously, according to this embodiment, maintenance of the storage may be improved. Overflowing of the storage may be avoided.

The sample-tube-storage-data may include expiration data of the at least one sample tube and/or the at least one tube rack. Based on these expiration data, the computing system may indicate via a display screen that the at least one sample tube is to be disposed of the at least one tube rack in which the at least one sample tube is placed and/or that the at least one tube rack is to be disposed of the storage in which the at least one tube rack is stored. For example, the display screen may display the expiration date of the biological sample contained in the at least one sample tube. The display screen may be comprised by the tube rack and/or the storage.

Further, the indicating may include prompting an alert that the at least one archived sample tube and/or the at least one stored tube rack is to be retrieved from the storage. The alert may be prompted on a display screen of the computing system and/or the apparatus. The prompting may also occur on a display screen being comprised by the at least one tube rack and/or the storage. The prompting the alert may include prompting the sample tube data, in particular the identifier of the at least one sample tube being expired, and/or the tube rack data, in particular the identifier of the at least one tube rack being expired and/or the location of the at least one sample tube being expired with respect to the at least one tube rack. The alert may be visual or audible or audio-visual.

In a specific embodiment, the storage may be connected to a lab automation system. When the storage capacity is over-exceeded, the at least one sample tube may be archived in a storage not connected to the lab automation system.

Advantageously, already existing storages may be used. No upgrades of the storage system are required.

In a specific embodiment, the storage may not be connected to a lab automation system. In other words, the storage may be operating independently of a lab automation system. That is the storage may already be preinstalled in a lab facility. Advantageously, also under these circumstances, the archiving method as described above is still applicable.

One or more steps of the archiving method may be performed repeatedly or may be performed several times. Additionally or alternatively, the one or more steps of the archiving method may be performed interchangeably. At least two of the method steps of the archiving method may also be performed simultaneously.

A further aspect of the present invention relates to a retrieving method for retrieving at least one sample tube containing a biological sample from at least one tube rack stored in a storage, in particular a cold storage, the method comprising the following steps:
receiving, by a computing system, a request for retrieving the at least one sample tube,
upon the request, obtaining, from a memory unit of the computing system, sample-tube-storage-data including sample tube data and/or tube rack data and/or storage data,
based on the sample-tube-storage-data, retrieving the at least one tube rack from the storage and retrieving the at least one requested sample tube from at least one receptacle, in particular in order to receive the at least one sample tube of the at least one retrieved tube rack, and
after retrieving the at least one requested sample tube, updating the sample-tube-storage-data.

Advantageously, the aforementioned aspect further facilitates sample tube tracking. It further renders sample tube tracking less time-consuming and more reliable. Moreover, it lowers the risk of patient misidentification. Errors in associating a sample with a particular patient may be reduced. Specifically, by retrieving the sample tube or after retrieving the sample tube, said sample tube may be received by the requestor, requesting retrieval of the sample tube. The sample tube may be retrieved for further processing of the sample and/or for disposing of the sample and/or the sample tube. In other words, after retrieving the sample tube from storage, the sample tube may be received at a dedicated recipient that specifically may be the requestor of the sample tube. At or by this recipient the sample and/or the sample tube may be further processed and/or discarded. It is also possible that requestor for the retrieval of the sample tube is different from the recipient. As an example, the computer system may automatically or upon request of a user initiate retrieval of the sample tube for disposal. This may be the case if the sample holding time has expired. Rather than providing the sample tube to the requestor, the sample tube may be received at a disposal unit or may be further processed for disposal.

All the features, steps, advantages and explanations provided with respect to the above-described aspect of an archiving method and its specific embodiments apply to this aspect of a retrieving method.

Both methods, the archiving method and the retrieving method as described below, may be combined to yield one method for managing sample tubes. The retrieving method may be performed subsequently to the archiving method. The archiving method may be performed subsequently to the retrieving method. Both the archiving method and the retrieving method may be standalone methods. The archiving method may be performed independently of the retrieving method. The retrieving method may be performed independently of the archiving method.

The method steps of the retrieving method may be performed as follows:
In a first step, the request to retrieve the at least one sample tube is received by the computing system. The request may be received at, on or via the computing system. A user may enter the request at the computing system, for example via a keyboard connected to the computing system and/or via a display screen of the computing system.

In a next step, the sample-tube-storage-data, in particular the data associated with the at least one sample tube to be retrieved, may be obtained from the memory unit of the computing system.

In a next step, the at least one sample tube may be retrieved from the at least one receptacle of the at least one tube rack being stored in the storage based on the sample-tube-storage-data. For example, the sample-tube-storage-data may be displayed on a display screen of the computing system as described above. Specifically, the location of the at least one tube rack in the storage and the at least one receptacle of the at least one tube rack from which the at least one sample tube is to be retrieved may be displayed. The computing system may also instruct a lab automation system, in particular a robotic arm, to retrieve the at least one sample tube based on the sample-tube-storage-data.

In a further next step, in particular a final step, the sample-tube-storage-data may be updated either manually by user input or automatically after the at least one requested sample tube has been retrieved. For example, the sample-tube-storage-data may be updated in such a way that the computing system deletes data associated with the at least one retrieved sample tube from the sample-tube-storage-data. Said data associated with the at least one retrieved sample tube may also be deleted manually by a user.

In a specific embodiment, the sample tube data may include at least one of an identifier of the at least one archived sample tube, sample tube expiration data and patient-related data. The identifier may include or be an identification number or identification code of the at least one sample tube. The sample tube expiration data may include the expiration date of the biological sample contained in the at least one sample tube and/or the number of tests or processes performed on the biological sample and/or the number of times the biological sample is to be used in tests or processes. The sample tube expiration data may also include information as to how long the at least one sample tube is utmost archived. The patient-related data may include an identification number or identification code associated with a specific patient. The patient-related data may also include biological identifiers such as blood type. The patient-related data may however not contain any information about the person of the patient. Additionally or alternatively, the tube rack data may include at least one of an identifier of the at least one stored tube rack, tube rack expiration data, patient-related data and occupation data of the at least one tube rack. The identifier may include or be an identification number or identification code of the at least one tube rack. The tube rack expiration data may include the expiration date of the biological sample contained in the at least one sample tube placed in the at least one tube rack and/or the number of tests or processes performed on said biological sample and/or the number of times the biological sample is to be used in tests or processes and/or the number of times the at least one tube rack is to be used. The tube rack expiration data may also include information as to how long the at least one tube rack is utmost stored in the storage. The patient-related data may include an identification number or identification code associated with a specific patient. The patient-related data may also include biological identifiers such as blood type. The patient-related data may however not contain any information about the person of the patient. The occupation data of the at least one tube rack may include information about which of the at least one receptacle is occupied with a sample tube or has received a sample tube. The information may include the number and/or location of the at least one occupied receptacle or of the at least one receptacle which has received a sample tube. The information may also include the coordinates with respect to the at least one tube rack of the at least one receptacle that has received a sample tube or is occupied with a sample tube. The information may also include the coordinates of the unoccupied receptacles, i.e. the receptacles which have not received a sample tube. The information may also include which of the receptacles of the at least one tube rack is occupied and which are unoccupied.

Additionally or alternatively, the storage data may include at least one of an identifier of the storage and a location of the at least one tube rack stored in the storage. The identifier may include or be an identification number or identification code of the storage. The location of the at least one tube rack stored in the storage may include the coordinates of the at least one tube rack stored in the storage. The coordinates may for example include the shelf number of the storage and the number or position of the at least one tube rack on said shelf.

Advantageously, according to this embodiment, sample tube tracking can be made more efficient, in particular the retrieving method can be carried out more efficiently.

In a specific embodiment, the method may further comprise the steps of: providing, by the computing system, the request to an apparatus being communicatively coupled with the computing system and configured to interact with the at least one tube rack before retrieving the at least one sample tube from the at least one tube rack, and visually indicating, based on the sample-tube-storage-data, the storage where the at least one tube rack is stored and/or the at least one tube rack from which the at least one sample tube is to be retrieved and/or the at least one receptacle of the at least one tube rack from which the at least one sample tube is to be retrieved.

Advantageously, according to this embodiment, the retrieval of a sample tube from a storage or an archive may be made more reliable. The chances of retrieving not the requested sample tube are reduced.

The method steps of this embodiment may be performed in the following order: providing and then visually indicating.

The apparatus may be configured as already described above. The at least one tube rack may also be configured as already described above or below.

The request, including, for example, information about the at least one sample tube to be retrieved such as the identifier of the at least one sample tube, may be provided to the apparatus via a wired or wireless network between the computing system and the apparatus. Providing the request to the apparatus may include sending all the information such as the sample tube data to identify the at least one requested sample tube to be retrieved to the apparatus. The apparatus may comprise a computing unit having a processor and a memory element for storing data.

The visually indicating may include, as already described above, that the computing system displays, via a display screen, the sample tube data and/or the tube rack data and/or the storage data to a user. The computing system may display, via the display screen, the location of the at least one sample tube to be retrieved with respect to the at least one tube rack and/or the location of the at least one tube rack having the at least one sample tube to be retrieved with respect to the storage in which the at least one tube rack is stored.

The visually indicating may additionally or alternatively include the computing system causing illuminating the at least one receptacle in which the at least one sample tube to be retrieved is placed, e.g. by a plurality of light emitting diodes integrated in the apparatus, and/or the at least one tube rack including said at least one receptacle and/or the location of the at least one tube rack in the storage. The computing device may cause the illuminating by at least one light emitting diode integrated in the at least one tube rack and/or the apparatus and/or the storage. The at least one light emitting diode may also be mounted to the apparatus and/or the at least one tube rack and/or the storage.

Exemplarily, the computing system may display, via the display screen, the at least one tube rack and/or the location of the at least one tube rack, in which the at least one sample tube to be retrieved is located, in the storage. Based on this display, a user may then retrieve the at least one tube rack from the storage and arrange it at or on the apparatus. Alternatively, a robotic arm of an automated lab system may retrieve the at least one tube rack from the storage and arrange it at or on the apparatus. Based on the sample-tube-storage-data and the request, the apparatus may illuminate the at least one receptacle of the at least one tube rack from which the at least one requested sample tube is to be retrieved either manually by a user or by a robotic arm.

The method may further include the step of placing the at least one retrieved tube rack in the apparatus before retrieving the at least one sample tube.

In a specific embodiment, the method may further comprise the steps of: arranging the at least one sample tube retrieved from the at least one tube rack at a scanning position of the apparatus, scanning, by the apparatus, the at least one sample tube when arranged at the scanning position with respect to the apparatus, and confirming, by the apparatus, based on the scan and the request, that the at least one requested sample tube has been retrieved.

Advantageously, a stored or archived sample tube can be retrieved from a storage or an archive even more reliably and with less errors.

The method steps of this embodiment may be performed in the following order: arranging, scanning and then confirming.

The apparatus may confirm, if the at least one scanned sample tube and the at least one requested sample tube correspond to each other. The confirmation may occur in form of a confirmation message displayed on a display screen of the apparatus and/or an audio signal and/or a visual signal. The audio signal may be a predetermined sound. The visual signal may be light of a predetermined colour being emitted by the apparatus.

In a specific embodiment, the computing system and/or the apparatus may comprise a display screen. The request for retrieving may be received via the computing system and/or the apparatus and displayed on the display screen of the computing system. Further, the computing system and/or the apparatus may comprise or be a mobile device, in particular a tablet PC or a smartphone or the like. In other words, a user of the computing system and/or the apparatus may manually provide a request for archiving of a sample. In one example, the request may be provided by means of a display screen, e.g. wherein the display screen is a touch sensitive display, that is a touch screen. In another example, the request may be provided using a physical keyboard and/or a mouse and/or voice commands. The above also applies for a request for retrieving a sample.

Advantageously, according to this embodiment, sample retrieving and thus sample tracking may be facilitated and rendered more user-friendly.

In a specific embodiment, the sample-tube-storage-data may be displayed on the display screen before retrieving the at least one sample tube and the at least one associated tube rack from the storage.

Advantageously, according to this embodiment, sample retrieving and thus sample tracking can be made even more reliable. It can be assured, by checking the display, that the correct sample tube and associated tube rack are retrieved from the storage.

One or more steps of the retrieving method may be performed repeatedly or may be performed several times. Additionally or alternatively, the one or more steps of the retrieving method may be performed interchangeably. At least two of the method steps of the retrieving method may also be performed simultaneously.

A further aspect of the present invention relates to a laboratory system including a computer system or being operatively coupled to a computer system which is adapted to carry out the archiving method and/or retrieving method as described above.

A further aspect of the present invention relates to a computer program product, in particular provided on a computer readable medium or implemented as a data stream, comprising computer executable instructions, which when executed by a computer processing device of a computer system of a laboratory system, cause the laboratory system to carry out the archiving method and/or retrieving method as described above.

A further aspect of the present invention relates to a tube rack for receiving at least one sample tube containing a biological sample, the tube rack comprising:
a body having a surface which is substantially planar in a horizontal direction, wherein the surface has at least one receptacle to receive the at least one sample tube, and
at least one optical indicator configured to visually indicate the at least one receptacle and/or the tube rack.

Advantageously, the above-mentioned tube rack renders sample tube holding and tracking, i.e. archiving and retrieving, more efficient. The tube rack can be easily, compactly and cost-efficiently manufactured.

All the features and explanations provided above with respect to the at least one tube rack, the at least one sample tube, the biological sample and the at least one receptacle also apply to the aspect of the present invention of a tube rack.

In this description, the terms "horizontal" and "vertical" refer to a three-dimensional, orthonormal xyz frame of reference being oriented with respect to earth ground.

The term "substantially" implies that manufacturing tolerances are included. For example, the substantially planar surface may have irregularities such as convexities and/or concavities. The convexities and/or concavities may not be visible by the human eye. The concavities and/or convexities may be on the scale of micrometres and/or nanometres. The substantially planar surface may also have some inclination with respect to the horizontal direction. The inclination may be in a range of -1 to 1 degrees.

The tube rack may be configured as described by the Applicant in European patent application No. 15 003 260.5, which is hereby incorporated by reference.

The body may be made from one piece. The body may comprise or consist of multiple pieces which are connectable or fixed to each other. The body may be formed of plastic. The body may be formed by injection molding. The body may be a cuboid, in particular a cube. The body may have plate shape.

The at least one receptacle may comprise or be an opening in the surface of the body. The at least one receptable may further comprise or be a clip for holding the at least one sample tube. The at least one receptacle may be formed in or at the surface such that the at least one sample tube, when placed in the at least one receptacle, may be held upright by the at least one receptacle. The at least one sample tube may be held in vertical direction by the at least one sample tube.

The at least one optical indicator may be arranged at the body of the tube rack. The at least one optical indicator may be arranged at the at least one receptacle. The at least one optical indicator may be integrated in or mounted to the tube rack, in particular to the body.

In a specific embodiment, the at least one optical indicator may be a display for displaying tube rack data and/or sample tube data. The tube rack data and/or the sample tube data may be specified as described above for the archiving method and/or the retrieving method. For example, the optical indicator may display the expiration date of the tube rack and/or of the at least one sample tube being placed in the tube rack, in particular of the biological sample contained in the at least one sample tube.

Advantageously, in this embodiment, sample tube holding and sample archiving as well as retrieving, and thus sample tracking, can be made even more efficient, in particular more reliable.

In a specific embodiment, the at least one optical indicator may be a light emitting diode. The at least one optical indicator may be arranged at the at least one receptacle such that light emitted by the light emitting diode may be visible from the at least one receptacle if no sample tube is present or placed in the at least one receptacle. The light emitting diode may also be disposed at the surface of the body.

Advantageously, in this embodiment, the tube rack can be manufactured cost-efficiently and it can easily be indicated from which receptacle a sample tube is to be retrieved or where a sample tube to be archived is to be placed.

The at least one optical indicator may be configured to indicate, in particular to display, information about the at least one tube rack such as occupation data of the at least one receptacle and/or indicate that the tube rack is to be archived by the above-mentioned archiving method according to the present invention or retrieved by the above-mentioned retrieving method according to the present invention. For example, the at least one optical indicator may indicate by one light emitting diode arranged at the body of the tube rack that the entire tube rack is to be archived or taken out of the storage. The indication may occur by turning on the light emitting diode or switching between light colours such as red and green.

In a specific embodiment, the at least one optical indicator may be powered inductively. Additionally or alternatively, the at least one optical indicator may be disposed at the body of the tube rack.

Advantageously, in this embodiment, the tube rack can be manufactured even more compactly and cost-efficiently. Furthermore, no internal power supply is required to provide the at least one optical indicator with power.

The body may contain one or more induction coils and an electric circuit so that, by applying an external magnetic field to the body of the tube rack, an electric current may be excited, the electric current powering the at least one optical indicator. For example, the shelves of a cold or cooled storage as mentioned above may be configured to generate a magnetic field which may induce an electric current in the tube rack when in contact with one of the magnetic shelves of the storage. Thereby, the at least one optical indicator may be turned on.

In the following, the drawings are described. The drawings depict one or more embodiments of the present invention. Individual features of the depicted embodiments may be combined to yield further embodiments. In the drawings, it is shown:
- **Fig. 1**: a flowchart of an archiving method according to an embodiment of the present invention;
- **Fig. 2**: a flowchart of a retrieving method according to an embodiment of the present invention;
- **Fig. 3**: an apparatus as used in an embodiment of the archiving and/or retrieving method according to the present invention;
- **Fig. 4**: an embodiment of a tube rack according to the present invention disposed at the apparatus of Fig. 3;
- **Fig. 5**: a mobile device with a display screen;
- **Fig. 6**: an operational diagram of a computing system used in an embodiment of the archiving and/or retrieving method according to the present invention; and
- **Fig. 7**: a schematic representation of an exemplary computing system and a schematic representation of an exemplary computing device.

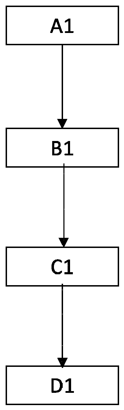

**Fig. 1** shows a flowchart of an embodiment of an archiving method according to the present invention.

When a sample tube associated with a specific patient is to be stored and archived, a user may enter, by means of a touch input, a corresponding request via a display screen of a tablet PC connected to the computing system, thereby the request is received by the computing system (step A1). The computing system may then indicate or display to the user via the tablet PC in which receptable of which tube rack the sample tube is to be placed and in which storage the tube rack is to be stored. Accordingly, the user then places the sample tube in the tube rack (step B1) and stores the tube rack in the corresponding storage (step C1). Finally, either the computing system records the location of the tube rack in the storage, the storage number, the tube rack number and the receptacle having the sample tube to be archived automatically in the memory unit of the computing system or the user records said information, i.e. the sample-tube-storage-data, manually in the memory unit by making a touch input on the display screen of the tablet PC of the computing system (step D1).

In another embodiment, when a sample tube is prepared by a lab automation system to be archived, the sample tube data may be logged into or send to the internal database of the computing system. This data record may contain: a tube rack unique identifier, e.g. RFID tag or sample tube number, and/or the position or location of the sample tube in the tube rack. When the tube rack is ready to be stored by the lab automation system or a user make such a decision by an input to the computing system, the future location of the tube rack storage, herein referred to as "storage data", may be indicated, e.g. by an on-screen notification "please store the rack in fridge n 2". Subsequently, the user may load the tube rack into the archiving location of the storage.

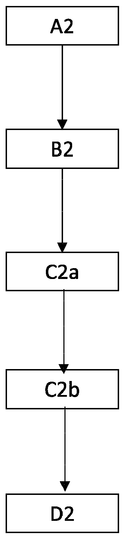

**Fig. 2** shows a flowchart of an embodiment of a retrieving method according to the present invention.

When a sample tube associated with a specific patient is needed, a user can make a corresponding request for retrieval via the display screen of the tablet PC connected to the computing system (step A2). For example, the user can enter the request by means of a touch input. The computing system may then, corresponding to the request, fetch the sample-tube-storage-data of the sample tube to be retrieved from its memory unit (step B2). The computing system may then display to the user on the display screen of the tablet PC the sample-tube-storage-data, i.e., for example, the number of the storage rack or fridge, the location or position of the tube rack holding the requested sample tube in the storage rack and the receptacle in which the requested sample tube is located. Based on this displayed information, the user may then retrieve the tube rack from the storage (step C2a) and retrieve the requested sample tube from the tube rack (step C2b). Finally, the computing system may recognize that the requested sample tube has been retrieved and updates automatically the sample-tube-storage-data in the memory unit or the user may manually update the sample-tube-storage-data by making a touch input on the display screen of the tablet PC of the computing system (step D2).

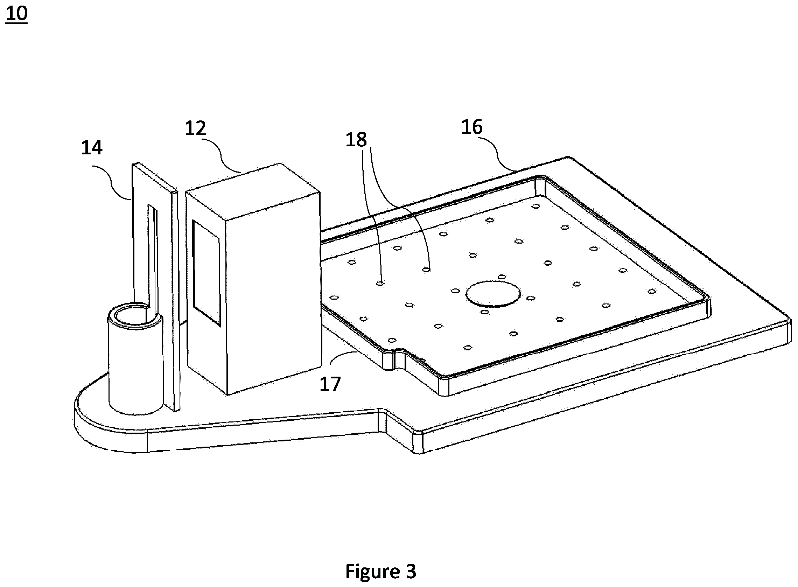

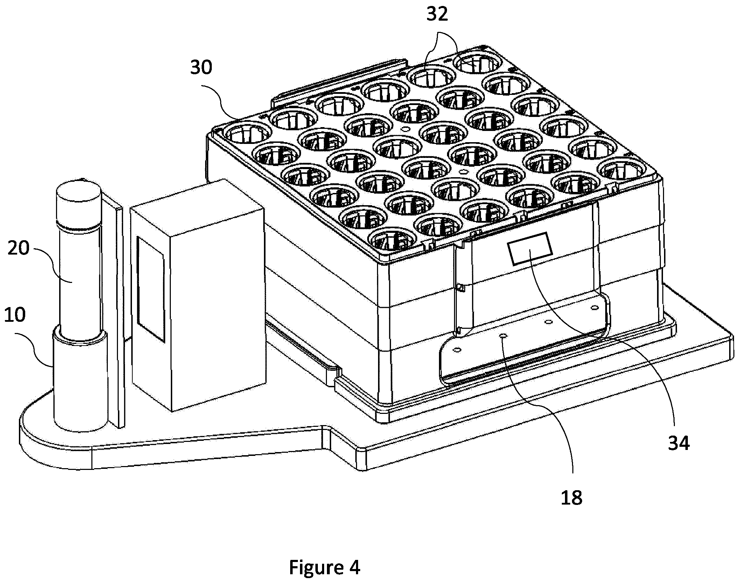

In another embodiment, when it is required to retrieve a sample tube, the user may search for the sample location in the data stored in the memory unit of the computing system which may indicate or display the location, such as fridge number or name, with the number of the tube rack, included in the storage data and tube rack data, in which the requested sample tube is located. The request for sample tube retrieval may be sent to the apparatus and waiting for further actions. If the apparatus is equipped with a display screen, there may be a pop-up message for the user. In the case that the display screen is the screen of a mobile device such as a tablet PC, the storage, in particular a storage room, may be located in different locations and being managed by other users. This may allow to notify the storage person to retrieve the tube rack containing the sample tube from the storage and send the sample tube, e.g. by means of pneumatics, to the core laboratory. When the tube rack is picked-up, it may be placed into the apparatus on top of a LED illuminated plate (see Fig. 3 below). The apparatus may indicate, by color LED light, the sample tube location in the tube rack. Accordingly, the sample tube may be picked by the user. The apparatus may recognize that the sample tube was picked and illuminates the scanning position or check-in station, e.g. a barcode reader spot, into which the user may place the sample tube for positive sample tube identification (see Fig. 4 below). When the barcode is read, the indicating light may change its color from red to green. Thereby, it is visually confirmed that the correct sample tube was picked and recognized. A user can then continue to proceed the sample tube. With a mobile device, there may be additional messages shown on the display screen of the mobile device like patient-related data. After the requested sample tube has been retrieved, the apparatus may send the information about the retrieval of the sample tube back to the computing system which may update the sample-tube-storage-data stored in the memory unit of the computing system accordingly.

**Fig.** 3 shows an apparatus 10 as used in an embodiment of the archiving and/or retrieving method according to the present invention. The apparatus 10 comprises a scanning unit 12, such as a barcode reader which is configured to read a barcode disposed at the sample tube, and a receiving unit 14 configured to receive a sample tube 20, when scanned by the scanning unit 12. In other words, when placed in the receiving unit 14, a sample tube 20 is in the scanning position with respect to the apparatus 10. The apparatus 10 further comprises a holding unit 16 which is configured to hold or receive a tube rack 30. The holding unit 16 is a receptacle formed by a perimeter 17 such that a tube rack 30 may be placed in the apparatus 10 by fittingly accommodating the tube rack 30 in the holding unit 16. Further, the apparatus 10 comprises a plurality of light emitting diodes (LEDs) 18 disposed in the bottom of the holding unit 16. The plurality of LEDs 18 is configured to indicate the presence and/or placement of sample tubes in the tube rack 30 when the tube rack 30 is placed in the holding unit 16. The plurality of LEDs 18 illuminates the receptacles 32 of the tube rack 30 from below. The apparatus 10 may also comprise an RFID reader or scanner (not visible) in the bottom of the holding unit 16, wherein the RFID reader or scanner is configured to read and/or scan and/or detect a RFID tag attached to or integrated in the body of the tube rack 30. The tube rack 30 is shown in Fig. 4.

**Fig. 4** shows an embodiment of a tube rack 30 according to the present invention disposed at the apparatus of Fig. 3. In Fig. 4, it is shown that the tube rack 30 comprising a plurality of receptacles 32 and an optical indicator 34 is placed in the holding unit 16 of the apparatus 10.

The optical indicator 34 may be a display that displays, for example, information about the tube rack and/or the contained sample tubes such as expiration dates of the biological samples. The optical indicator may also comprise or be a light emitting diode which indicates if the tube rack 30 is placed in the apparatus 10 or if the tube rack 30 is to be picked up from the storage. The tube rack 30 may also have multiple optical indicators which are disposed at the top surface of the body of the tube rack 30, where the receptacles 32 are located, and indicate the receptacles 32 in which the sample tube is to be placed and/or from which a sample tube is to be taken. However, Fig. 4 only shows one optical indicator 34 which is disposed at or in a lateral side of the body of the tube rack 30.

According to this embodiment, the one or more optical indicators 34 may comprise or consist of a digital display. The one or more optical indicators 34 may e.g. illuminate in specific colors thereby indicating different states of the rack. As an example, the one or more optical indicators 34 may illuminate red indicating that the rack is at the wrong position and/or not correctly positioned and/or is to be retrieved from the archive, e.g. because one or more samples are overdue. In addition or alternatively, the one or more optical indicators 34 may illuminate in green, indicating that the rack is properly located in the archive and/or correct storage of all the samples. Other colors and color combinations may be possible.

In addition or alternatively, the one or more optical indicators 34 may also display data, such as sample-tube-storage data. Specifically, the one or more optical indicators 34 may indicate which receptacle 32 contains which sample tube and/or may display from which receptacle 32 a sample tube may be retrieved and/or in which receptacle 32 a sample tube may be placed. For example, it is possible that the one or more optical indicators 34 display the closest expiration date of the samples stored in the sample tubes located in the tube rack 30. It is also possible that the one or more optical indicators 34 display for some or all sample tubes their location in the tube rack 30, e.g. by means of grid coordinates of the receptacle 32. In addition or alternatively, it is possible that the one or more optical indicators 34 display, for some or all sample tubes the expiration date of the sample. As an alternative or in addition, for one or more sample tubes the archiving date may be displayed.

In addition or alternatively, it is possible that the one or more optical indicators 34 display, for some or all sample tubes their content, that is which sample is contained in a respective sample tube.

Specifically, it is possible that the one or more optical indicators 34 provide both color codes as well as display data such as sample-tube-storage-data.

The plurality of LEDs 18 and the receptacles 32 are arranged in the holding unit 16 of the apparatus 10 and in the body of the tube rack 30 such that, when the tube rack 30 is placed in the apparatus 10, the plurality of LEDs 18 and the receptacles 32 are aligned. This alignment allows for illuminating, by the apparatus 10, particular receptacles 32 from which a user is to take a sample tube. In other words, after a tube rack 30 is placed e.g. within perimeter 17 of the holding unit 16 in one embodiment, each LED 18 corresponds to a respective receptacle 32. Thus, in this embodiment advantageously it is possible that for a sample tube to be placed in a dedicated receptacle 32 the corresponding LED 18 may be illuminated, specifically using a preselected operation color of this LED 18. Therefore, it is possible that the receptacle 32 can be distinguished from other receptacles 32 illuminated in a different color or no color at all. A user or a robot may be guided by the light and/or the color of the light in order to place the tube in the correct receptacle 32. Advantageously this results in the archiving process being easier to be carried out while at the same time being more reliable. Specifically, according to this embodiment, the risk of placing a sample tube in the wrong receptacle 32 can be highly reduced.

Also, during retrieval of a tube from the receptacle 32 the corresponding LED 18 may be illuminated. Since there may be space between a wall of the receptacle 32 and the tube, the light emitted from the LED 18 still may be visible from the top of the rack 30, even if the sample tube contains a fluid generally absorbing or blocking the light and even if the tube cap absorbs or blocks the light emitted from the LED 18. In other words, light emitted from the LED 18 does not need to penetrate the tube in order to be visible from above the rack 30.

In another embodiment, it is possible that at the same process step tubes may be retrieved from the rack 30 and tubes may be placed in the rack 30. The corresponding receptacles 32 may be illuminated simultaneously or subsequently. The corresponding receptacles 32 may be illuminated with the same color or different colors. As an example, a receptacle 32, from which a tube should be retrieved may be illuminated via an LED 18 emitting light of one color, such as red. A receptacle 32, in which a tube should be added may be illuminated via an LED 18 emitting light of a different color, such as green.

The tube rack 30 comprises a lateral opening which conventionally is used for a user to grip and/or hold the rack 30. In Fig. 4 a number of the plurality of LEDs 18 is visible through the lateral opening.

When retrieving a tube from the rack 30, subsequently to discarding the tube from the receptacle 32, the user or a robot can put the particular sample tube 20 into the receiving unit 14 in front of the scanning unit 12 of the apparatus for double confirmation that the requested sample tube was picked. By putting the sample tube 20 into the receiving unit 14, the sample tube 20 is arranged in the scanning position with respect to the apparatus 10. For example, the scanning unit 12 may be a barcode reader for reading a barcode attached to the sample tube 20 or a RFID reader for reading a RFID label or tag attached to the sample tube 20 for confirmation. In other words, by scanning the sample tube 20, it is assured that the scanned sample tube 20 corresponds to the requested sample tube either for archiving or retrieving.

A similar process step may be applied, when archiving a tube. The tube may be placed in the receiving unit 14 and identified in the receiving unit 14, e.g. by scanning a label on the tube or reading an RFID chip attached to or embedded in the tube. Upon identification of the tube, a receptacle 32 in which the tube should be placed may be illuminated by a corresponding LED 18. Thus, the user or a robot may place the tube in the correct receptacle 32 based on the illumination by the LED 18.

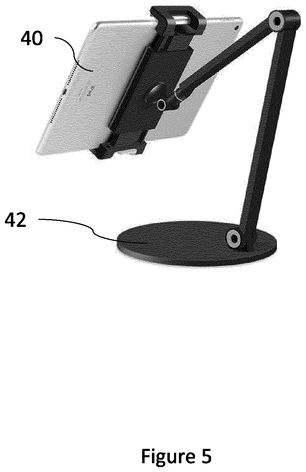

**Fig. 5** shows a mobile device 40 with a display screen. The mobile device is attached to a device holder 42. The mobile device 40 may be a standalone device such as a tablet PC. The mobile device 40 may be wirelessly connected to the computing system. Preferably, the mobile device 40 is integrated in or mounted to the apparatus 10. The display screen of the mobile device 40 may be integrated in the apparatus 10.

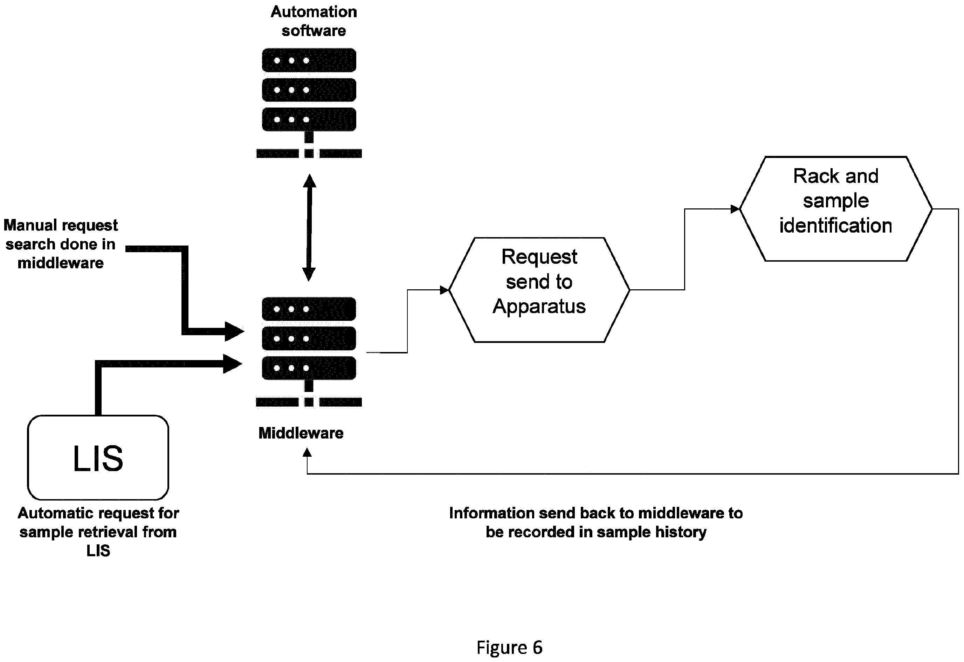

**Fig. 6** shows a diagram of how the computing system operates in archiving and/or retrieving a sample tube.

Specifically, Fig. 6 represents the flow of the data and orders in the above described process. By way of example, Fig. 6 shows a possible IT structure of a laboratory setup like a laboratory information system (LIS), a middleware, and an automation software. The automation software may be responsible for managing the track system. The middleware receives the request for sample retrieval and/or sample archiving. The request may be obtained as a manual request, e.g. of a user providing one or more sample tubes arranged in a tube rack for archiving said one or more sample tubes. The user may provide the request for archiving or retrieval via a graphical user interface displayed on a display device. The display device may be a display device of or connected to storage for archiving sample tubes. The display device may be a display device of a device of the user connected to the middleware e.g. through a local network or the internet. The device of the user may be a desktop PC or a mobile device such as a laptop PC, a mobile tablet device, a smartphone, etc. The user may provide the request for archiving or retrieval of one or more sample tubes using the graphical user interface displayed on the display device.

Alternatively, the request may be obtained by the LIS providing one or more sample tubes arranged in a tube rack for archiving said one or more sample tubes or requesting retrieval of one or more sample tubes stored in the archive.

The middleware forwards the request to an apparatus allowing and/or carrying out the sample tube(s) retrieval and/or archiving, that is the sample tube retrieval and more specifically the retrieval of the rack carrying the sample tube(s) and/or archiving of the sample tube(s) located in the tube rack.

Upon sample retrieval and/or archiving, feedback is provided to the middleware. The middleware updates the sample history, that is the sample-tube-storage-data is updated. The middleware may host a database for storing the sample-tube-storage data. In addition or alternatively, the middleware may be connected to a cloud storage and the sample-tube-storage data may be stored in the cloud storage.

Also, the middleware may update the LIS and/or a user providing the information on one or more of: the storage of the sample tube, the retrieval of the sample tube, the date of the storage of the sample tube, the date of the retrieval of the sample tube, the location of the sample tube, the location of the tube rack, etc. The update to the user may be by way of a display and/or providing a digital message, such as an email, a text message, etc. The update to the user may include printing of a receipt containing one or more of: confirmation of storing and archiving the sample tube(s), location of the sample tube(s), confirmation of retrieving the sample tube(s), etc.

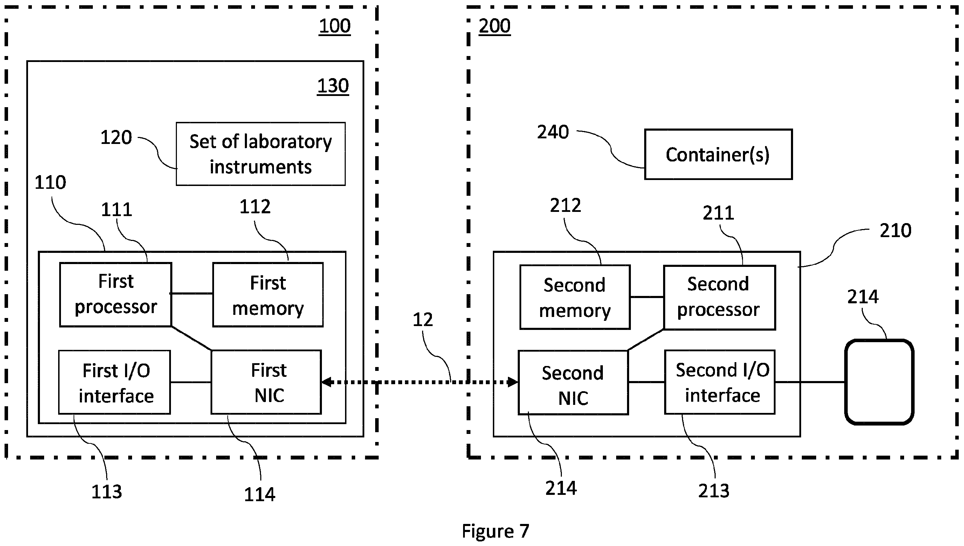

**Fig. 7** shows a schematic representation of a computing system 110 and a schematic representation of a computing device 210.

The computing system 110 comprises a processor 111 (e.g. a CPU, a GPU, or the like), and a memory 112. The processor 111 is configured to carry out at least one step of the archiving and/or retrieving method as described above. The memory 112 may comprise a primary memory and a secondary memory (not shown). In particular, the secondary memory stores a computer program comprising instructions which when executed by the processor 111 cause the computing system 110 to carry out at least one step of the archiving and/or retrieving method. The computing system 110 may also comprise an input output (I/O) interface 113 for communicating with input/output units, such as a display screen, a keyboard, a touch screen, a printer, or the like.

The computing device 210comprises a processor 211 (e.g. a CPU, a GPU, or the like), and a memory 212. The processor 211 is configured to carry out at least one step of the archiving and/or retrieving method as described above. The memory 212 may comprise a primary memory and a secondary memory (not shown). In particular, the secondary memory stores a computer program comprising instructions which, when executed by the processor 211, cause the computing device 210 to carry out at least one step of the archiving and/or retrieving method as described above. The computing device 210 comprises an input output (I/O) interface 113 for communicating with an input/output unit, e.g. in the form of a display screen 214. In particular, the computing device 211 may be a laptop or a tablet PC or a smartphone. If the computing device 211 is a tablet PC, the display screen 214 is in particular a touch screen. The apparatus 10 may comprise or be the computing device 211.

The computing system 110 and the computing device 210 comprise a first Network Interface Controller (NIC) 114 and a second NIC 214, respectively, configured to connect said system and device with one or more networks (e.g. an intranet, the internet, a cellular network, or the like). The computing system 110 and the computing device 210 may exchange data with one another via the first NIC 114 and the second NIC 214 by using a protocol suite such as TCP or IP, said protocol being schematically represented in Fig. 7 by the double-headed dashed line 12.

The computing system 110 is comprised in an Automated Laboratory System (ALS) 130 that comprises one or more laboratory instruments (not shown) of a set of laboratory instruments 120. The ALS 130 may comprise transporting means (not shown), e.g. tracks, belts and/or tube containers, or the like, for transporting tubes comprising biological samples to the laboratory instruments for testing. In particular, the computing system 110 is configured to control the laboratory instruments and the transporting means of the ALS 130. The ALS 130 is located in a laboratory 100.

The computing device 210 is accessible to and usable by a user such as a laboratory agent (not shown) which, in this example, is a laboratory operator, e.g. a human being trained to operate laboratory devices such as diagnostic analyzers, pre-analytical and post-analytical laboratory systems. The computing device 210 is located in the laboratory operator's office 200 or in a storage room 200 or another laboratory 200.

Alternatively, the laboratory agent may be a laboratory device (not shown), e.g. a sample handling robot configured to manipulate and transport biological samples.

Biological samples are placed in a container or sample tube 240, which is subsequently brought to the laboratory 100. The laboratory agent may have a plurality of containers 240 at their disposal and, optionally, a plurality of tube racks configured to accommodate one or more containers 240. The tube racks are used for safely and correctly transporting the containers 240. The laboratory agent further has the computing device 210 at their disposal, which is configured to interface with a user or the laboratory agent e.g. via the display screen 214.

The laboratory 100 receives the biological sample in the container or sample tube and analyses it, e.g. by using the ALS 130. The computing system 110 and the computing device 210 are distinct and may be separated by a physical distance that ranges e.g. from the order of metres to the order of kilometres. For example, the laboratory operator's office 200 may be located on site at the laboratory, in which case the computing system 110 and the computing device 210 are locally connected, e.g. by means of a LAN. In another example, the laboratory operator's office 200 may be at a physician's office (where clinical tests are prescribed) that is remote from the laboratory, in which case the computing system 110 and the computing devices 210 may communicate remotely over the Internet.

The laboratory 100 and the room 200 may coincide or be the same. The computing system 110 may comprise or be a server. The computing system 110 may comprise or be a cloud computing platform or a distributed computer system. The computing system 110 may comprise or be one or more desktop PCs or workstations which are in communication with each other by means of a network connection, e.g. via the internet and/or LAN and/or Wi-Fi.

In conclusion, by providing the present invention, sample tube tracking may be improved. The present invention renders sample tube tracking less time-consuming and more reliable. Furthermore, it lowers the risk of patient misidentification. Errors in associating a sample with a particular patient may be reduced.

### List of Reference Numerals

- 10: apparatus
- 12: scanning unit
- 14: receiving unit
- 16: holding unit
- 18: plurality of LEDs
- 20: sample tube
- 30: tube rack
- 32: plurality of receptacles
- 34: optical indicator
- 40: mobile device
- 42: device holder

## Claims

1. An archiving method for archiving in a storage, in particular in a cold storage, at least one sample tube (20) containing a biological sample using at least one tube rack (30), the method comprising the following steps:
receiving (A1), by a computing system, a request for archiving the at least one sample tube (20),
placing (B1) the at least one sample tube (20) in at least one receptacle (32) of the at least one tube rack (30) to receive the at least one sample tube (20),
recording (D1) and/or updating, in a memory unit of the computing system, sample-tube-storage-data including sample tube data and/or tube rack data and/or storage data, and
storing (C1) the at least one tube rack (30) in the storage.

2. The method according to claim 1, wherein the sample tube data include at least one of an identifier of the at least one archived sample tube, sample tube expiration data and patient-related data, and/or
wherein the tube rack data include at least one of an identifier of the at least one stored tube rack, tube rack expiration data, patient-related data and occupation data of the at least one tube rack, and/or
wherein the storage data include at least one of an identifier of the storage and a location of the at least one tube rack stored in the storage.

3. The method according to any one of the preceding claims, wherein the method further comprises:
arranging the at least one sample tube (20) to be archived at a scanning position of an apparatus (10) being communicatively coupled with the computing system and configured to interact with the at least one tube rack (30) before the at least one tube rack (30) is stored in the storage,
scanning, by the apparatus (10), the at least one sample tube (20) when arranged at the scanning position with respect to the apparatus (10),
visually indicating, based on the scan and the request, the at least one tube rack (30) to be stored and/or the at least one receptacle (32) of the at least one tube rack (30) in which the at least one sample tube (20) is to be placed and/or the storage where the at least one tube rack (30) is to be stored,
wherein the computing system may optionally receive user input as to a location in the storage where the at least one tube rack (30) is stored and/or the computing system may optionally receive user input to record the sample-tube-storage-data.

4. The method according to any one of the preceding claims, wherein the computing system and/or the apparatus (10) comprises a display screen, and the request for archiving is received via the computing system and/or the apparatus (10) and displayed on the display screen of the computing system, wherein the computing system is optionally a mobile device (40), in particular a tablet PC.

5. The method according to claim 3 or 4, wherein the visually indicating includes:
illuminating the at least one tube rack (30) to be stored and/or the at least one receptacle (32) of the at least one tube rack (30) in which the at least one sample tube (20) is to be placed and/or the storage where the at least one tube rack (30) is to be stored, and/or
displaying the sample tube data and/or the tube rack data and/or the storage data on a display screen.

6. The method according to any one of the preceding claims, wherein the method further comprises:
indicating, by the computing system, based on the sample-tube-storage-data, that the at least one archived sample tube (20) and/or the at least one stored tube rack (30) and/or the biological sample contained in the at least one archived sample tube (20) is expired, and/or the at least one archived sample tube (20) and/or the at least one stored tube rack (30) is to be retrieved from the storage,
wherein the indicating optionally includes:
prompting an alert that the at least one archived sample tube (20) and/or the at least one stored tube rack (30) is to be retrieved from the storage.

7. A retrieving method for retrieving at least one sample tube (20) containing a biological sample from at least one tube rack (30) stored in a storage, in particular a cold storage, the method comprising the following steps:
receiving (A2), by a computing system, a request for retrieving the at least one sample tube (20),
upon the request, obtaining (B2), from a memory unit of the computing system, sample-tube-storage-data including sample tube data and/or tube rack data and/or storage data,
based on the sample-tube-storage-data, retrieving (C2a) the at least one tube rack (30) from the storage and retrieving (C2b) the at least one requested sample tube (20) from at least one receptacle (32), and
after retrieving the at least one requested sample tube (20), updating (D2) the sam ple-tube-storage-data.

8. The method according to claim 7, wherein the sample tube data include at least one of an identifier of the at least one archived sample tube, sample tube expiration data and patient-related data, and/or
wherein the tube rack data include at least one of an identifier of the at least one stored tube rack, tube rack expiration data, patient-related data and occupation data of the at least one tube rack, and/or
wherein the storage data include at least one of an identifier of the storage and a location of the at least one tube rack stored in the storage.

9. The method according to any one of claims 7 to 8,
wherein the method further comprises:
providing, by the computing system, the request to an apparatus (10) being communicatively coupled with the computing system and configured to interact with the at least one tube rack (30) before retrieving the at least one sample tube (20) from the at least one tube rack (30), and
visually indicating, based on the sample-tube-storage-data, the storage where the at least one tube rack (30) is stored and/or the at least one tube rack (30) from which the at least one sample tube (20) is to be retrieved and/or the at least one receptacle (32) of the at least one tube rack (30) from which the at least one sample tube (20) is to be retrieved.

10. The method according to claim 9,
wherein the method further comprises: positioning the at least one tube rack (30) on a holding unit (16) of the apparatus (10), wherein the visually indicating includes illuminating at least one of the receptacles (32) of the tube rack (30) positioned on the apparatus (10) by at least one light emitting diode; and/or
wherein the method further comprises:
arranging the at least one sample tube (20) retrieved from the at least one tube rack (30) at a scanning position of the apparatus (10),
scanning, by the apparatus (10), the at least one sample tube (20) when arranged at the scanning position with respect to the apparatus (10), and
confirming, by the apparatus (10), based on the scan and the request, that the at least one requested sample tube (20) has been retrieved.

11. The method according to any one of claims 9 to 10, wherein the computing system and/or the apparatus (10) comprises a display screen and the request for retrieving is received via the display screen of the computing system, wherein the computing system is optionally a a mobile device (40), in particular a tablet PC, wherein the sample-tube-storage-data is optionally displayed on the display screen before retrieving the at least one sample tube (20) from the storage.

12. A laboratory system including a computer system or being operatively coupled to a computer system which is adapted to carry out the method of any one of the claims 1 to 6 and/or the method of any one of the claims 7 to 11.

13. A computer program product, in particular provided on a computer readable medium or implemented as a data stream, comprising computer executable instructions, which when executed by a computer processing device of a computer system of a laboratory system, cause the laboratory system to carry out the method according to any one of claims 1 to 6 and/or the method according to any one of claims 7 to 11.

14. A tube rack (30) for receiving at least one sample tube (20) containing a biological sample, the tube rack comprising:
a body having a surface which is substantially planar in a horizontal direction, wherein the surface has at least one receptacle (32) to receive the at least one sample tube (20), and
at least one optical indicator (34) configured to visually indicate the at least one receptacle (32) and/or the tube rack (30).

15. The tube rack (30) according to claim 14, wherein the at least one optical indicator (34) is a display for displaying tube rack data and/or sample tube data; and/or
wherein the at least one optical indicator (34) is a light emitting diode; and/or
wherein the at least one optical indicator (34) is powered inductively; and/or
wherein the at least one optical indicator (34) is disposed at the body of the tube rack (30).

## Amended claims

### Amended claims in accordance with Rule 137(2) EPC.

1. An archiving method for archiving in a storage, in particular in a cold storage, at least one sample tube (20) containing a biological sample using at least one tube rack (30), the method comprising the following steps:
receiving (A1), by a computing system, a request for archiving the at least one sample tube (20),
arranging the at least one sample tube (20) to be archived at a scanning position of an apparatus (10) being communicatively coupled with the computing system and configured to interact with the at least one tube rack (30) before the at least one tube rack (30) is stored in the storage,
scanning, by the apparatus (10), the at least one sample tube (20) when arranged at the scanning position with respect to the apparatus (10),
visually indicating, based on the scan and the request and by means of at least one optical indicator disposed at the body of the at least one tube rack, the at least one tube rack (30) to be stored and/or at least one receptacle (32) of the at least one tube rack (30) in which the at least one sample tube (20) is to be placed,
placing (B1) the at least one sample tube (20) in the at least one receptacle (32) of the at least one tube rack (30) to receive the at least one sample tube (20),
recording (D1) and/or updating, in a memory unit of the computing system, sample-tube-storage-data including sample tube data and/or tube rack data and/or storage data, and
storing (C1) the at least one tube rack (30) in the storage.

2. The method according to claim 1, wherein the sample tube data include at least one of an identifier of the at least one archived sample tube, sample tube expiration data and patient-related data, and/or
wherein the tube rack data include at least one of an identifier of the at least one stored tube rack, tube rack expiration data, patient-related data and occupation data of the at least one tube rack, and/or
wherein the storage data include at least one of an identifier of the storage and a location of the at least one tube rack stored in the storage.

3. The method according to any one of the preceding claims, wherein the method further comprises:
visually indicating, based on the scan and the request, the storage where the at least one tube rack (30) is to be stored,
wherein the computing system may optionally receive user input as to a location in the storage where the at least one tube rack (30) is stored and/or the computing system may optionally receive user input to record the sample-tube-storage-data.

4. The method according to any one of the preceding claims, wherein the computing system and/or the apparatus (10) comprises a display screen, and the request for archiving is received via the computing system and/or the apparatus (10) and displayed on the display screen of the computing system, wherein the computing system is optionally a mobile device (40), in particular a tablet PC.

5. The method according to claim 3 or 4, wherein the visually indicating includes:
illuminating the at least one tube rack (30) to be stored and/or the at least one receptacle (32) of the at least one tube rack (30) in which the at least one sample tube (20) is to be placed and/or the storage where the at least one tube rack (30) is to be stored, and/or
displaying the sample tube data and/or the tube rack data and/or the storage data on a display screen.

6. The method according to any one of the preceding claims, wherein the method further comprises:
indicating, by the computing system, based on the sample-tube-storage-data, that the at least one archived sample tube (20) and/or the at least one stored tube rack (30) and/or the biological sample contained in the at least one archived sample tube (20) is expired, and/or the at least one archived sample tube (20) and/or the at least one stored tube rack (30) is to be retrieved from the storage,
wherein the indicating optionally includes:
prompting an alert that the at least one archived sample tube (20) and/or the at least one stored tube rack (30) is to be retrieved from the storage.

7. A retrieving method for retrieving at least one sample tube (20) containing a biological sample from at least one tube rack (30) stored in a storage, in particular a cold storage, the method comprising the following steps:
receiving (A2), by a computing system, a request for retrieving the at least one sample tube (20),
upon the request, obtaining (B2), from a memory unit of the computing system, sample-tube-storage-data including sample tube data and/or tube rack data and/or storage data,
providing, by the computing system, the request to an apparatus (10) being communicatively coupled with the computing system and configured to interact with the at least one tube rack (30) before retrieving the at least one sample tube (20) from the at least one tube rack (30),
visually indicating, based on the sample-tube-storage-data and by means of at least one optical indicator disposed at the body of the at least one tube rack, the at least one tube rack (30) from which the at least one sample tube (20) is to be retrieved and/or the at least one receptacle (32) of the at least one tube rack (30) from which the at least one sample tube (20) is to be retrieved
based on the sample-tube-storage-data, retrieving (C2a) the at least one tube rack (30) from the storage and retrieving (C2b) the at least one requested sample tube (20) from at least one receptacle (32), and
after retrieving the at least one requested sample tube (20), updating (D2) the sample-tube-storage-data.

8. The method according to claim 7, wherein the sample tube data include at least one of an identifier of the at least one archived sample tube, sample tube expiration data and patient-related data, and/or
wherein the tube rack data include at least one of an identifier of the at least one stored tube rack, tube rack expiration data, patient-related data and occupation data of the at least one tube rack, and/or
wherein the storage data include at least one of an identifier of the storage and a location of the at least one tube rack stored in the storage.

9. The method according to any one of claims 7 to 8,
wherein the method further comprises:
visually indicating, based on the sample-tube-storage-data, the storage where the at least one tube rack (30) is stored.

10. The method according to claim 9,
wherein the method further comprises: positioning the at least one tube rack (30) on a holding unit (16) of the apparatus (10), wherein the visually indicating includes illuminating at least one of the receptacles (32) of the tube rack (30) positioned on the apparatus (10) by at least one light emitting diode; and/or
wherein the method further comprises:
arranging the at least one sample tube (20) retrieved from the at least one tube rack (30) at a scanning position of the apparatus (10),
scanning, by the apparatus (10), the at least one sample tube (20) when arranged at the scanning position with respect to the apparatus (10), and
confirming, by the apparatus (10), based on the scan and the request, that the at least one requested sample tube (20) has been retrieved.

11. The method according to any one of claims 9 to 10, wherein the computing system and/or the apparatus (10) comprises a display screen and the request for retrieving is received via the display screen of the computing system, wherein the computing system is optionally a a mobile device (40), in particular a tablet PC, wherein the sample-tube-storage-data is optionally displayed on the display screen before retrieving the at least one sample tube (20) from the storage.

12. A laboratory system including a computer system or being operatively coupled to a computer system which is adapted to carry out the method of any one of the claims 1 to 6 and/or the method of any one of the claims 7 to 11.

13. A computer program product, in particular provided on a computer readable medium or implemented as a data stream, comprising computer executable instructions, which when executed by a computer processing device of a computer system of a laboratory system, cause the laboratory system to carry out the method according to any one of claims 1 to 6 and/or the method according to any one of claims 7 to 11.

14. A tube rack (30) for receiving at least one sample tube (20) containing a biological sample, the tube rack comprising:
a body having a surface which is substantially planar in a horizontal direction, wherein the surface has at least one receptacle (32) to receive the at least one sample tube (20), and
at least one optical indicator (34) configured to visually indicate the at least one receptacle (32) and/or the tube rack (30),
wherein the at least one optical indicator (34) is disposed at the body of the tube rack (30).

15. The tube rack (30) according to claim 14, wherein the at least one optical indicator (34) is a display for displaying tube rack data and/or sample tube data; and/or
wherein the at least one optical indicator (34) is a light emitting diode; and/or
wherein the at least one optical indicator (34) is powered inductively.
